# EUROPEAN PATENT APPLICATION

(11) **EP 2 332 589 A2**
(43) Date of publication of application: **15.06.2011**
(21) Application number: 10194555.8
(22) Date of filing: 10.12.2010
(51) Int. Cl.: A61L 27/30, A61L 27/32

(54) **Methods for coating implants**

(30) Priority: 11.12.2009 US 636379
(71) Applicant: Biomet Manufacturing Corp., Warsaw, IN 46582 (US)
(72) Inventor: Kumar, Mukesh, Warsaw, IN 46582 (US); Gupta, Gautam, Waraw, IN 46582 (US); Ronk, Robert, Pierceton, IN 46562 (US)
(74) Representative: Morawski, Birgit

(57) **Abstract**

An implant and method for applying an osteoconductive coating on a non-conductive surface of an implant. The method includes depositing an electroconductive interlayer on at least a portion of a non-conductive implant surface. A secondary process is applied to the interlayer and an osteoconductive coating is thereby formed on the implant. In various embodiments, the electroconductive interlayer is deposited as a non-structural film and comprises a dense, non-porous metal such as titanium, titanium alloys, cobalt, cobalt alloys, chromium, chromium alloys, tantalum, tantalum alloys, iron alloys, stainless steel, and mixtures thereof. The osteoconductive coating may include a metal, a porous metal, or calcium phosphate. The osteoconductive coating may include additional agents, such as bone product, growth factor, bioactive agent, antibiotic, or combinations thereof.

## Description

### INTRODUCTION

The present technology relates to non-conductive medical implants provided with conductive or osteoconductive coatings, and methods of their manufacture.

In the growing field of medical devices, there is a continued need to provide lightweight orthopedic implants having enhanced in-growth capability. It is known that the time between in-growth of an implant until the full mechanical loadability is achieved can be reduced if the bone-contacting surface of the implant has a surface comprising a calcium phosphate phase such as hydroxyapatite. Although implants containing polymers and ceramics, including implants having a surface comprising polymers and ceramics, have many advantages, there remains a need to increase their osteoconductivity to provide enhanced implants.

### SUMMARY

The present technology provides an implant comprising a substrate defining a non-conductive surface. An electroconductive interlayer is deposited on at least a portion of the non-conductive surface, and an osteoconductive coating is applied on at least a portion of the electroconductive interlayer. The electroconductive interlayer comprises a non-structural film having an average thickness of less than about 3 µm.

The present technology also provides methods for applying an osteoconductive coating on a non-conductive surface of an implant. In various embodiments, the method includes depositing an electroconductive interlayer on at least a portion of a non-conductive surface of the implant. An osteoconductive coating is then applied on the interlayer using an electrical deposition process. In various aspects, the electroconductive interlayer is deposited as a non-structural film and comprises a dense, non-porous metal such as titanium, titanium alloys, cobalt, cobalt alloys, chromium, chromium alloys, tantalum, tantalum alloys, iron alloys, stainless steel, and mixtures thereof.

In certain embodiments, the method for applying an osteoconductive coating on a non-conductive surface of an implant comprises depositing a non-structural metallic interlayer having an average thickness of less than about 3 µm on at least a portion of the non-conductive surface of the implant. A secondary metallic layer is then applied onto the interlayer using a porous plasma spray technique, thereby forming an osteoconductive coating on the implant. In various embodiments, the secondary metallic layer comprises a titanium alloy.

The present technology also provides methods for applying an osteoconductive coating on a non-conductive surface of an implant comprising a porous scaffold. The method includes depositing a non-structural metal interlayer coating on at least a portion of the porous scaffold using a vapor deposition technique. An osteoconductive coating is subsequently formed on the interlayer, having discrete regions of calcium phosphate on the implant. In various embodiments, the porous scaffold comprises a polymer and the metal interlayer comprises silver.

### DRAWINGS

Figure 1 is an exemplary acetubular cup shaped medical implant having osteoconductive coating attached thereto; and

Figure 2 is a cross section of Figure 1 taken along the line 2-2.

It should be noted that the figures set forth herein are intended to exemplify the general characteristics of materials, methods and devices among those of the present technology, for the purpose of the description of certain embodiments. These figures may not precisely reflect the characteristics of any given embodiment, and are not necessarily intended to define or limit specific embodiments within the scope of this technology.

### DETAILED DESCRIPTION

The following description of technology is merely exemplary in nature of the subject matter, manufacture and use of one or more inventions, and is not intended to limit the scope, application, or uses of any specific invention claimed in this application or in such other applications as may be filed claiming priority to this application, or patents issuing therefrom. A non-limiting discussion of terms and phrases intended to aid understanding of the present technology is provided at the end of this Detailed Description.

The present technology relates to methods for improving the osteo-compatability of medical implants. As such, the present technology encompasses a wide variety of therapeutic and cosmetic applications, in human or other animal subjects, and the specific materials and devices used must be biomedically acceptable. As used herein, such a "biomedically acceptable" component is one that is suitable for use with humans and/or animals without undue adverse side effects (such as toxicity, irritation, and allergic response) commensurate with a reasonable benefit risk/ratio.

The present technology provides implants, and methods for applying an osteoconductive coating(s) on a non-conductive surface of implants. Implants include those comprising: a substrate having a non-conductive surface; an electroconductive interlayer disposed on at least a portion of the non-conductive surface; and an osteoconductive coating disposed on at least a portion of the electroconductive interlayer, wherein the electroconductive interlayer comprises a non-structural film between the portion of the non-conductive surface and the osteoconductive coating, the interlayer having an average thickness of less than about 3 µm.

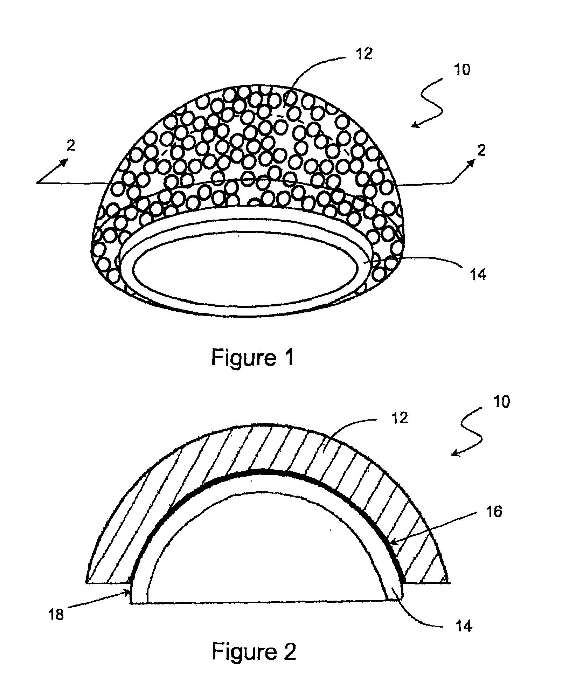

Figure 1 illustrates an exemplary acetubular cup medical implant 10 having an osteoconductive coating 12 deposited on a substrate 14. Figure 2 is a cross sectional view of Figure 1, taken along the line 2-2 and illustrates an intermediate interlayer 16 between the substrate 14 of the implant 10 and the osteoconductive coating 12. The implant 10 has a substrate 14 and a surface 18, and comprises one or more materials. In some embodiments, the implant 10 comprises a substrate 14 that provides the non-conductive surface 18, wherein the substrate 14 and non-conductive surface 18 8 have an identical or essentially identical composition. In other embodiments, the surface 18 and the substrate 14 may comprise similar or identical materials, but have varying compositions, wherein (for example) the concentration of a component of the substrate 14 varies from the core of the substrate to the surface 18. In other embodiments, the implant 10 may comprise a substrate 14 comprising one material, and a non-conductive surface 18 comprising a different material, for example, as a discrete layer on the substrate.

The non-conductive surface 18 (and, in some embodiments, the substrate 14) consists of a material which is substantially non-conductive. As used herein, the terms "substantially non-conductive" or "non-conductive" refer to a surface that is not capable of conducting an electrical current under conditions that are suitable to effect application of an osteoconductive coating 12 in the processes of the present technology. It will be understood by one of ordinary skill in the art that, in various embodiments, non-conductive surfaces comprise materials which, as comprised in the implants, are not effective to conduct a current sufficient to allow electrical deposition of an osteoconductive coating under industrially-acceptable conditions. The conductive nature of the surface will vary according to such factors as the specific materials making up the surface, their concentrations, and the specific electrical deposition process and conditions to be used. Such materials include ceramics, polymers, and mixtures thereof.

Ceramics used in a ceramic-containing implant can be made of any suitable biomedically acceptable ceramic material. Generally, such ceramic materials include inorganic, non-metallic materials that are processed or consolidated at a high temperature. In various embodiments, ceramic materials may include oxides, nitrides, borides, carbides, and sulfides. More specifically, ceramics useful herein include titanium oxide, titanium dioxide, alumina ceramics, zirconia ceramics, stabilized zirconia, silicon carbide, metal nitride, metal carbide, and dopants, mixtures, and combinations thereof.

Polymers used in a polymer-containing implant can be made of any suitable biomedically acceptable polymer. Generally, such polymers may include polyethylene, cross-linked polyethylene (XLPE), polyetheretherketone (PEEK), carbon fiber reinforced PEEK (CF-PEEK), polyamide, polyurethane, polytetrafluoroethylene (PTFE), ultra high molecular weight polyethylene (UHMWPE), carbon fiber reinforced UHMWPE (CF-UHMWPE), LactoSorb®, polymers of lactide, glycolide, and caprolactone (for example, poly(L-lactic acid) (PLLA), poly(D,L-lactide), poly(lactic acid-co-glycolic acid), 50/50 (DL-lactide-co-glycolide), polydioxanone, polycaprolactone and co-polymers and mixtures thereof such as poly(D,L-lactide-co-caprolactone)), resorbable polymers, copolymers and mixtures thereof. In various embodiments, PEEK or UHMWPE may be specifically desirable because of their respective mechanical properties, biocompatibility, and potentially low wear rates when used as, or in connection with, a bearing surface.

The ceramic or polymer substrate 14, or body, is typically provided with a shape operable as a medical implant for a human subject. In certain embodiments, the implant may be further shaped or formed after one or more osteoconductive coating is applied thereon. The medical implant can be an orthopedic implant, for example, an acetubular cup as shown in Figures 1 and 2, a knee implant such as a condyle implant, a shoulder implant, a spinal implant, a bone fixation device, bone plate, spinal rod, rod connector, femoral resurfacing system, spacer, scaffold, and the like. The medical implant can also be custom made or a generic shape for filling in a bone defect caused by surgical intervention or disease. In certain embodiments, the implant comprises a porous scaffold substrate, such as a porous polymer scaffold. As used herein, "implant" may refer to an entire implant as a whole, or a portion thereof; portions may be as large as necessary or as small as areas of an individual attachment screw or fastening component. For example, in certain embodiments, the implant may comprise one or more resorbable polymer screws. It may be desirable to provide the coatings of the present technology to one or more thread regions of the screw or fastening component for increased bone in-growth. The metallic layer(s) of the coating may also assist with the physical insertion of the implant, for example, a thin metal coating on the outermost thread regions may create a "self-tapping" type fastening component.

The implant can also be attached as part of an orthopedic insert, such as those disclosed in U.S. Patent Application Serial No. 12/038,570 filed February 27, 2008 and published as U.S. Patent Application Publication No. 2008/0147187, Bollinger et al., published June 19, 2008, which is incorporated by reference herein in its entirety. The implant can also be used to form a geostructure, which is a three-dimensional geometric porous engineered structure that is self supporting and is constructed of rigid filaments joined together to form regular or irregular geometric shapes. The structure is described in more detail in U.S. Patent No. 6,206,924, Timm, issued March 27, 2001 which is incorporated by reference.

Methods of the present technology comprise depositing an electroconductive interlayer 16 on at least a portion of a substantially non-conductive implant substrate or surface 18. Such an interlayer 16 may also be specifically directed to include coating any pore walls. The interlayer coating 16 may be continuous and cover the entire surface 18 of the substrate 14, or optionally only a portion or specific region thereof. In various embodiments, the electroconductive interlayer 16 is deposited as metal or metallic layer, and in particular, it may be deposited as a thin, non-structural, metallic film. As used herein, the term "non-structural" means that is does not substantially add mechanical strength, integrity, or enhance other structural properties of the substrate, as compared to a substrate without such a film layer. It is envisioned that a non-structural film of the present technology principally assists in making the implant surface conductive in order to be compatible with various electrodeposition and plasma spray techniques, as will be described in more detail below.

The electroconductive interlayer can be applied having variable properties, such as thickness, texture, porosity, density, and the like. In various embodiments, the electroconductive interlayer may comprise a dense, non-porous metal such as titanium, titanium alloys, cobalt, cobalt alloys, chromium, chromium alloys, tantalum, tantalum alloys, iron alloys, stainless steel, and mixtures thereof. In certain embodiments, the interlayer may comprise an antimicrobial material, such as carbon, silver, platinum, and mixtures thereof. Generally, the electroconductive interlayer is uniformly deposited having an average thickness, i.e., normal to the substrate surface, of less than about 3 µm. In various embodiments, the electroconductive interlayer is deposited having an average film thickness of from about 0.1 µm to about 3 µm. In certain embodiments, the electroconductive interlayer is deposited having an average film thickness of from about 0.1 µm to about 2 µm, in other aspects it may be provided from about 2 µm to about 3 µm, or having a thickness of about 2.5 µm. In still other embodiments, the coating may have an average thickness greater than 3 µm. The electroconductive interlayer may cover small substrate imperfections, while not enhancing other mechanical properties. In certain other embodiments, the electroconductive layer may include, mimic, or resemble the same or similar surface features as the substrate, as may be desired.

The electroconductive interlayer may be applied using conventional thermal spraying, plasma spray coating, sputtering and ion beam deposition (IBD) processes, physical vapor deposition (PVD) techniques, and chemical vapor deposition (CVD) techniques as are known in the art. Specialized techniques such as ion beam enhanced deposition (IBED) may also be used to generate the interlayer of the present technology. U.S. Patent No. 5,055,318, Deutchman et al., issued October 8, 1991 provides various process details and is incorporated by reference herein. Surface texturing may also be performed as a pretreatment, prior to coating, but is not required. Surface texturing may include various mechanical, chemical, or optical modifications. Generally, components are thoroughly cleaned or placed in an ultrasonic bath prior to being coated. In certain embodiments, the interlayer may be applied using solution based methods, including casting and dipping. It should be understood that when using methods that involve high temperature, the temperature, as well as other parameters of the particular process, should be commensurate with the substrate properties. For example, temperature should be monitored with regard to any potential for modifying the properties of the plastic or ceramic substrate.

In regard to the various antimicrobial embodiments, a conductive interlayer coating of silver or carbon can likewise be achieved using any suitable physical vapor deposition method. Silver can also be applied using solution precipitation techniques, while carbon can also be applied using appropriate chemical vapor deposition techniques. A system to apply the electroconductive interlayer may be similar to that used in scanning electron microscopy (SEM), where a substrate is placed in a plasma field of silver, carbon, or other desired material. The charged ions, e.g., silver or carbon, are attracted to the implant substrate due to the difference in electrical potential.

Once the electroconductive interlayer is deposited on at least a portion of the implant substrate, a secondary treatment can then be performed on the interlayer using an electrical deposition process, thereby forming an osteoconductive coating on the implant. Electrical deposition processes may include porous plasma spray and electrodeposition techniques. Accordingly, the osteoconductive coating may include a secondary metallic layer, such as a porous titanium alloy, or it may include a non-metallic component, such as calcium phosphate or hydroxyapatite. Notably, the various antimicrobial and antibacterial embodiments disclosed herein are generally not compromised or otherwise affected after a subsequent hydroxyapatite coating is applied because the underlying silver, carbon, platinum, or other interlayer material is not affected in the deposition process.

In various embodiments, the secondary treatment provides a coating resulting from porous plasma spray treatment. For example, a component having the electroconductive interlayer can be placed in a suitable plasma spray system where the interlayer surface is plasma sprayed with a desired metal powder, such as Ti6Al4V powder, to generate a rough osteo-integrating surface. For implants requiring an articulating surface, portions may be polished to an acceptable roughness, for example less than about 10 nm Ra, to provide the articulating surface. In various other embodiments, the secondary treatments, or electrical deposition processes, can provide an osteoconductive coating comprising at least one of a metal, a porous metal, such as a porous metal matrix or a porous plasma sprayed metal, and calcium phosphate regions. The osteoconductive coating can also include bone products, growth factors, bioactive agents, antibiotics, and combinations thereof.

The present technology is not limited to providing a coating having one or two layers. For example, calcium phosphate phases (CPP) can be deposited directly to the interlayer, or can be further applied onto a secondary metallic layer to improve bioinert behavior of implant materials. Calcium phosphate phases boast a lot of bioactive potential, thus enabling chemical bonding to natural bone. As used herein, the main inorganic constituent CPP may contain amorphous calcium phosphate (Ca₉(P0₄)₆ • nH₂O), hydroxyapatite (Ca₁₀(PO₄)₆(OH)₂), octacalcium phosphate (Ca₈H₂ (PO)₆H₂O), or brushite (CaHPO₄ • 2H₂O), or mixtures thereof. The CPP can additionally be doped with ions such as fluoride, silver, magnesium, carbonate, strontium, or sodium.

As is known in the art, hydroxyapatite (Ca₁₀(PO₄)₆(OH)₂) is a CPP biocompatible material that is similar in composition to the major inorganic mineral content of natural bone. As such, hydroxyapatite coatings provided on metallic or otherwise electroconductive medical implants can enhance an implant's s osteoconductivity potential, among other things. Common deposition techniques for coating hydroxyapatite onto implants can include plasma spray coating, electrochemical deposition, and sol-gel deposition. One advantage of using a deposition process can be that it is not a "line of sight" process and thus can provide a complete coating coverage of complex shaped substrates. While plasma spray coating is a widely used method, its high process conditions (high temperature) can result in coating properties that deviate from the mineral phase of bone, especially with respect to crystal structure and solubility. For example, the high process temperatures may cause partial decomposition of hydroxyapatite, resulting in the formation of other CPP, including amorphous calcium phosphate (ACP), α-tricalcium phosphate (TCP), β-TCP, tetracalcium phosphate, and calcium oxide. Numerous studies have found that more mechanical failure occurs at the metal/hydroxyapatite interface, rather than at the bone/hydroxyapatite interface. Accordingly, electrochemical deposition techniques are advantageous.

In various embodiments of the present technology, after the electroconductive interlayer coating is provided, including at least one area having an electroconductive surface, an electrochemical deposition can be carried out in an electrolysis cell, or bath, in which the implant, as least the metallic or otherwise electroconductive interlayer, is cathodically polarized. For example, a three-electrode arrangement can be made including a calomel electrode used as a reference electrode, a platinum sheet or gauze used as the counter electrode, and the interlayer, or optional secondary metallic layer of the implant, is provided as the cathode. The deposition process can take place biomimetically, near physiological pH and temperature conditions.

At least a portion of the electroconductive surface is placed in contact with an electrolyte solution comprising calcium ions and phosphate ions. For example, the electrolyte can comprise a Ca²⁺/HₓPO₄^{(3-×)-} containing solution. In various embodiments, the ratio of the concentration of the calcium ions and the phosphate ions is chosen such that it is equal or at least equivalent to their concentrations in hydroxyapatite. The electrolyte solution may include calcium chloride, calcium chloride dihydrate, or calcium acetate as the source of calcium ions, and ammonium dihydrogen phosphate as the source of phosphate ions. The choice of the particular salts used may be based on availability. In certain embodiments, the electrolyte solution is prepared using solutions that correspond to a final concentration of calcium:phosphate of about 5:3. Of course it is also possible to choose a different concentration ratio of calcium and phosphate ions, if desired. In various embodiments, collagen fibers may additionally be dispersed into the electrolyte solution. In these instances, beneficial collagen fibers are incorporated into the osteoconductive coating as the calcium phosphate regions are being formed.

Once contact has been established between the electroconductive or metallic surface of the implant and the electrolyte solution, for example, by immersing the implant into an electrolyte bath, an electrical potential is applied between the electroconductive surface and the electrolyte solution. It should be understood that the actual current density required may vary for each different type of component processed, and may depend upon the total surface area and surface finish, as well as the power sources available.

Electrochemical deposition of a calcium phosphate phase depends in part on the pH-dependent solubility of the calcium phosphate, which has been found to decrease with increasing pH. Using an electrochemical deposition process, one can control the pH at the cathode/electrolyte interface.

In certain embodiments, the electrochemical deposition process may be carried out by cathodic polarization in a number of successive, repeated process cycles. For example, a process cycle may include cathodic polarization in one or more successive steps, in certain embodiments during at least two discrete intervals of time, with identical or different (increased or decreased) constant current densities, and a rinsing and/or drying phase following thereon.

In various embodiments, optional agents can be coated onto or in a surface of the osteoconductive coating component of the implant. For example, a coated implant may additionally be placed in an antibiotic solution where it adsorbs at least one antibiotic or other optional material into the osteoconductive coating. Further optional materials may include bone materials, blood products, bioactive materials, additional ceramics, polymers, and combinations thereof. Bone materials include bone powder and demineralized bone. Blood products include blood fractions and other blood derived materials, such as platelet rich plasma. Bioactive agents useful herein include organic molecules, proteins, peptides, peptidomimetics, nucleic acids, nucleoproteins, antisense molecules, polysaccharides, glycoproteins, lipoproteins, carbohydrates and polysaccharides, botanical extracts, and synthetic and biologically engineered analogs thereof, living cells such as stem cells (e.g., adipose derived stem cells) chondrocytes, bone marrow cells, viruses and virus particles, natural extracts, and combinations thereof. Specific examples of bioactive materials include hormones, antibiotics and other antiinfective agents, hematopoietics, thrombopoietics, agents, antiviral agents, antiinflammatory agents, anticoagulants, therapeutic agents for osteoporosis, enzymes, vaccines, immunological agents and adjuvants, cytokines, growth factors, cellular attractants and attachment agents, gene regulators, vitamins, minerals and other nutritionals, nutraceuticals and combinations thereof. Additional ceramics include resorbable or non-resorbable ceramic materials, such as glasses or ceramics comprising mono-, di-, tri-, α-tri-, β-tri-, and tetra-calcium phosphate, calcium sulfates, calcium oxides, calcium carbonates, magnesium calcium phosphates, phosphate glass, bioglass, and mixtures thereof. Additional polymers include resorbable or non-resorbable polymers, such as polyhydroxyalkanoates, polylactones and their copolymers. In various embodiments, the optional material may be a resorbable ceramic, resorbable polymer, antimicrobial, demineralized bone, blood product, stem cell, growth factor or mixture thereof. Preferably, the optional material(s) assist or facilitate in-growth of new tissue into the medical implant.

The embodiments described herein are exemplary and not intended to be limiting in describing the full scope of compositions and methods of the present technology. Equivalent changes, modifications and variations of embodiments, materials, compositions and methods can be made within the scope of the present technology, with substantially similar results.

### Non-limiting Discussion of Terminology:

The headings (such as "Introduction" and "Summary") and subheadings used herein are intended only for general organization of topics within the present disclosure, and are not intended to limit the disclosure of the technology or any aspect thereof. In particular, subject matter disclosed in the "Introduction" may include novel technology and may not constitute a recitation of prior art. Subject matter disclosed in the "Summary" is not an exhaustive or complete disclosure of the entire scope of the technology or any embodiments thereof. Classification or discussion of a material within a section of this specification as having a particular utility is made for convenience, and no inference should be drawn that the material must necessarily or solely function in accordance with its classification herein when it is used in any given composition.

The description and specific examples, while indicating embodiments of the technology, are intended for purposes of illustration only and are not intended to limit the scope of the technology. Moreover, recitation of multiple embodiments having stated features is not intended to exclude other embodiments having additional features, or other embodiments incorporating different combinations of the stated features. Specific examples are provided for illustrative purposes of how to make and use the compositions and methods of this technology and, unless explicitly stated otherwise, are not intended to be a representation that given embodiments of this technology have, or have not, been made or tested.

As used herein, the words "desirable", "preferred" and "preferably" refer to embodiments of the technology that afford certain benefits, under certain circumstances. However, other embodiments may also be preferred or desirable, under the same or other circumstances. Furthermore, the recitation of one or more preferred or desired embodiments does not imply that other embodiments are not useful, and is not intended to exclude other embodiments from the scope of the technology.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

Although the open-ended term "comprising," as a synonym of non-restrictive terms such as including, containing, or having, is used herein to describe and claim embodiments of the present technology, embodiments may alternatively be described using more limiting terms such as "consisting of" or "consisting essentially of." Thus, for any given embodiment reciting materials, components or process steps, the present technology also specifically includes embodiments consisting of, or consisting essentially of, such materials, components or processes excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein.

As referred to herein, all compositional percentages are by weight of the total composition, unless otherwise specified. Disclosures of ranges are, unless specified otherwise, inclusive of endpoints. Thus, for example, a range of "from A to B" or "from about A to about B" is inclusive of A and of B. Disclosure of values and ranges of values for specific parameters (such as temperatures, molecular weights, weight percentages, etc.) are not exclusive of other values and ranges of values useful herein. It is envisioned that two or more specific exemplified values for a given parameter may define endpoints for a range of values that may be claimed for the parameter. For example, if Parameter X is exemplified herein to have value A and also exemplified to have value Z, it is envisioned that Parameter X may have a range of values from about A to about Z. Similarly, it is envisioned that disclosure of two or more ranges of values for a parameter (whether such ranges are nested, overlapping or distinct) subsume all possible combination of ranges for the value that might be claimed using endpoints of the disclosed ranges. For example, if Parameter X is exemplified herein to have values in the range of 1 - 10, or 2 ^{_} 9, or 3 ^{_} 8, it is also envisioned that Parameter X may have other ranges of values including 1 ^{_} 9, 1 ^{_} 8, 1 ^{_} 3, 1 - 2,2 ^{_}10,2 ^{_} 8,2 ^{_} 3,3 ^{_} 10, and 3 ^{_} 9.

When an element or layer is referred to as being "on", "engaged to", "connected to" or "coupled to" another element or layer, it may be directly on, engaged, connected or coupled to the other element or layer, or intervening elements or layers may be present. In contrast, when an element is referred to as being "directly on", "directly engaged to", "directly connected to" or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

## Claims

1. A method for applying an osteoconductive coating on a non-conductive surface of an implant, the method comprising:
a. depositing an electroconductive interlayer on at least a portion of the non-conductive surface of the implant; and
b. applying an osteoconductive coating on the interlayer using an electrical deposition process.

2. The method of Claim 1, wherein the electroconductive interlayer comprises a dense, non-porous metal selected from the group consisting of titanium, titanium alloys, cobalt, cobalt alloys, chromium, chromium alloys, tantalum, tantalum alloys, iron alloys, stainless steel, and mixtures thereof.

3. The method of Claims 1 or 2, wherein the electroconductive interlayer is deposited as a non-structural film having an average film thickness of less than about 3 µm, preferably from about 2 µm to about 3 µm.

4. The method of any of Claims 1 through 3, wherein the electroconductive interlayer comprises an antimicrobial material selected from the group consisting of silver, carbon, platinum, and mixtures thereof.

5. The method of any of Claims 1 through 3, wherein the non-conductive implant surface comprises a polymer selected from the group consisting of PEEK, polyamide, polyurethane, PTFE, UHMWPE, resorbable polymers, and copolymers and mixtures thereof.

6. The method of any of Claims 1 through 3, wherein the non-conductive implant surface comprises a ceramic selected from the group consisting of alumina, zirconia, metal nitride, metal carbide, and mixtures thereof.

7. The method of any of Claims 1 through 6, wherein the non-conductive implant comprises a porous polymer scaffold.

8. The method of Claim 1, wherein applying the osteoconductive coating comprises:
a. contacting at least a portion of the electroconductive interlayer with an electrolyte solution comprising calcium ions and phosphate ions; and
b. applying an electrical potential between the electroconductive interlayer and the electrolyte solution, thereby forming calcium phosphate regions on the electroconductive interlayer.

9. The method of Claim 8, further comprising dispersing collagen fibers into the electrolyte solution and incorporating the collagen fibers into the osteoconductive coating as the calcium phosphate regions are formed.

10. The method of Claim 1, wherein the osteoconductive coating comprises a material selected from the group consisting of metals, calcium phosphate, and mixtures thereof.

11. The method of Claim 10, wherein the osteoconductive coating comprises a porous metal.

12. The method of Claim 10, wherein the osteoconductive coating further a material selected from the group consisting of bone materials, blood products, bioactive agents, and combinations thereof.

13. The method of Claim 12, further comprising adsorbing an antibiotic into the osteoconductive coating by placing the implant in a solution of the antibiotic.

14. The method of Claim 1, wherein the step of depositing the electroconductive interlayer onto the substrate comprises coating the substrate with a thin film metallic layer using a process selected from the group consisting of ion beam deposition, physical vapor deposition, chemical vapor deposition, and plasma spray coating, preferably an ion beam deposition process.

15. A method of Claim 1 for applying an osteoconductive coating on a non-conductive surface of an implant comprising a porous scaffold, the method comprising:
a. depositing a non-structural metal interlayer coating on at least a portion of the porous scaffold using a vapor deposition technique; and
b. forming an osteoconductive coating on the interlayer having discrete regions of calcium phosphate.

16. The method of Claim 15, wherein the porous scaffold comprises a polymer and the metal interlayer comprises silver.

17. An implant, comprising:
a substrate having a non-conductive surface;
an electroconductive interlayer disposed on at least a portion of the non-conductive surface; and
an osteoconductive coating disposed on at least a portion of the electroconductive interlayer,
wherein the electroconductive interlayer comprises a non-structural film between the portion of the non-conductive surface and the osteoconductive coating, the interlayer having an average thickness of less than about 3 µm.

18. The implant according to Claim 17, wherein the electroconductive interlayer has an average thickness from about 2 µm to about 3 µm.

19. The implant according to Claim 17, wherein the substrate comprises a porous scaffold and the osteoconductive coating comprises calcium phosphate.
